# EUROPEAN PATENT APPLICATION

(11) **EP 2 468 850 A1**
(43) Date of publication of application: **27.06.2012**
(21) Application number: 10809959.9
(22) Date of filing: 17.08.2010
(51) Int. Cl.: C12N 5/071, C12N 5/07

(54) **CELL PRESERVATION METHOD**

(30) Priority: 19.08.2009 JP 2009190083
(71) Applicant: Takara Bio, Inc., Otsu-shi Shiga 520-2193 (JP)
(72) Inventor: TANABE, Masashige, Otsu-shi, Shiga 520-2193 (JP); ISONO, Tomomi, Otsu-shi, Shiga 520-2193 (JP); ENOKI, Tatsuji, Otsu-shi, Shiga 520-2193 (JP)
(74) Representative: Vossius & Partner
(86) International application number: PCT/JP2010/063850
(87) International publication number: WO 2011/021618

(57) **Abstract**

A method for cryopreservation of cells **characterized by** the use of a solution containing, as essential components, a sodium salt, a potassium salt, a saccharide, a cryoprotectant, and a hydrogencarbonate salt and/or a carbonate salt, the solution further optionally containing a component selected from the group consisting of proteins, magnesium salts, and calcium salts. According to the present invention, a method for cryopreservation of cells capable of maintaining a high viability rate after thawing, and a cryopreservation solution used in the cryopreservation are provided. In addition, by using the method for cryopreservation, the cells can be preserved in a state of maintaining a high viability rate even after thawing, and also maintaining their physiological functions; further, a cell washing step after freezing and thawing is unnecessary, thereby making it very useful in the fundamental researches of cells and the application studies on the medical treatment.

## Description

### TECHNICAL FIELD

The present invention relates to a method for cryopreservation of cells, and a cryopreservation solution for use in the method.

### BACKGROUND ART

In recent years, with the advancement of medicine, fundamental studies and treatments using live cells have been widely performed. Therefore, a technique for preserving the cells safely while maintaining activity of the cells has been necessitated, and various studies have been made thereon. As a method for preserving cells, in a case of a short-term preservation, a method for preserving cells in a suspension state without freezing has been known (for example, Patent Publication 1); or in a case of a long-term preservation, a method for preserving the cells including freezing has been known (for example, Patent Publication 2). For example, in adoptive immunotherapy or donor lymphocyte infusion (DLI) therapy, which is an immunotherapy against cancer, there are some cases where it would be operably difficult to use (administer) the cells immediately after the preparation; therefore, it is needed to cryopreserve the cells for a certain period until the cells are used. As a method for cryopreservation, a method in which a solution prepared by adding a cryoprotectant to a cell culture medium comprising about 40 kinds of ingredients is used as a cell preservation solution has been known.

However, the conventional method uses a medium containing about 40 kinds of ingredients as mentioned above, and there is a possibility of including those undesired for cells or a live body among these ingredients. Further, in a case where cells are administered to a live body after thawing the cryopreserved cells, it is necessary to preserve the cells in a solution state for a period of time until the cells are administered to the live body, but there is a problem in the conventional cryopreservation solution that a viability rate of cells, in other words, the number of live cells, is markedly lowered with the passage of the preservation time. In addition, in a case where the step of washing the cells is necessary after thawing, there is also a problem that the viability rate of the cells is further lowered.

### PRIOR ART REFERENCES

### PATENT PUBLICATIONS

Patent Publication 1: Japanese Patent Laid-Open No. 2006-230396
Patent Publication 2: Japanese Patent Laid-Open No. 2002-233356

### SUMMARY OF THE INVENTION

### PROBLEMS TO BE SOLVED BY THE INVENTION

An object of the present invention is to provide a method for cryopreservation of cells capable of maintaining a high viability rate, and a cryopreservation solution having essential components for use in the cryopreservation.

### MEANS TO SOLVE THE PROBLEMS

In order to solve the above problems, the present inventors have verified every one of vast combinations from about 40 kinds of ingredients in the medium that are conventionally used in cryopreservation of cells in view of finding components that are harmless to a live body and essential upon the cryopreservation of the cells. As a result, the present inventors have found that cells having a high viability rate even after thawing are obtained by using a solution containing a sodium salt, a potassium salt, a saccharide, a cryoprotectant, and a hydrogencarbonate salt and/or a carbonate salt. Further, by the use of the solution, even when the above-mentioned cryopreserved cells are thawed and subjected to a long-term preservation in a liquid suspension state, the present inventors have surprisingly found that it is possible to preserve the cells, while maintaining a high viability rate even though the cells are once frozen, and the present invention has been perfected thereby.

Specifically, the present invention, in sum, relates to:
[1] a method for cryopreservation of cells characterized by the use of a solution containing, as essential components, a sodium salt, a potassium salt, a saccharide, a cryoprotectant, and a hydrogencarbonate salt and/or a carbonate salt, the solution further optionally comprising a member selected from the group consisting of proteins, magnesium salts, and calcium salts;
[2] the method according to [1], wherein the saccharide is at least one saccharide selected from the group consisting of glucose, galactose, fructose, mannose, xylose, and arabinose;
[3] the method according to [1], wherein the cryoprotectant is at least one protectant selected from the group consisting of dimethyl sulfoxide, hydroxyethyl starch, ethylene glycol, and glycerol;
[4] the method according to [1], wherein the protein is a protein originated from blood;
[5] a method for preservation of cells **characterized in that** the method comprises thawing a cell solution subjected to cryopreservation in accordance with the method as defined by [1], and further preserving the cell solution in a liquid suspension state; and
[6] a cryopreservation solution of cells, **characterized in that** the cryopreservation solution contains, as essential components, a sodium salt, a potassium salt, a saccharide, a cryoprotectant, and a hydrogencarbonate salt and/or a carbonate salt, and that the solution further optionally contains a member selected from the group consisting of proteins, magnesium salts, and calcium salts.

### EFFECTS OF THE INVENTION

According to the present invention, the cryopreservation for obtaining cells maintaining a high viability rate after thawing is made possible, and further even in a case where the cells are preserved in a liquid suspension state after thawing, the cells maintain a high viability rate, and also maintain physiological functions; therefore, the present invention is very useful in the fundamental studies of cells, and applied studies and applications to medicine.

### BRIEF DESCRIPTION OF THE DRAWINGS

[Figure 1] A diagram showing one example of cell viability activity when essential components of a solution used in the method for preservation of the present invention are studied.
[Figure 2] A diagram comparing cell viability activities after repeatedly freezing and thawing the cryopreserved cells.
[Figure 3] A diagram comparing cell viability activities in a case where the cells are preserved in a liquid suspension state for a long period of time after thawing the cryopreserved cells.
[Figure 4] A diagram comparing cell viability activities in a case where the cells are preserved in a liquid suspension state for a long period of time after thawing the different cells which are cryopreserved.
[Figure 5] A diagram comparing cell viability activities after repeatedly freezing and thawing the cryopreserved cells.
[Figure 6] A diagram comparing cell viability activities in a case where the cells are preserved in a liquid suspension state for a long period of time after thawing the cryopreserved cells.

### MODES FOR CARRYING OUT THE INVENTION

The present invention will be described more specifically hereinbelow.

The present invention provides a method for cryopreservation of cells, **characterized in that** the method includes suspending cells in a solution containing a sodium salt, a potassium salt, a saccharide, a cryoprotectant, and a hydrogencarbonate salt and/or a carbonate salt, and subjecting the resulting cell suspension to cryopreservation. In other words, the sodium salt, the potassium salt, the saccharide, the cryoprotectant, and the hydrogencarbonate salt and/or the carbonate salt are necessary and sufficient components for cryopreservation of cells in the present invention [also referred to as essential components of a solution used in the method for cryopreservation of the present invention (a cryopreservation solution of the present invention)].

The "sodium salt" as used herein is not particularly limited so long as the sodium salt generates sodium ions when dissolved in a solvent, and an oxoacid salt, a halide, an oxide, a hydroxide, an inorganic salt, an organic acid salt, or the like can be used. For example, the oxoacid acid includes sodium percarbonate (Na₂CO₄), sodium dithionite (sodium dithionite, sodium hydrosulfite) (Na₂S₂O₄), sodium sulfite (Na₂SO₃), sodium hydrogensulfite (NaHSO₃), sodium sulfate (sodium sulfate (generic name): Na₂SO₄), sodium thiosulfate(hypo) (Na₂S₂O₃), sodium nitrite (NaNO₂), and sodium nitrate (NaNO₃); the halide includes sodium fluoride (NaF), sodium chloride (NaCl), sodium bromide (NaBr), and sodium iodide (NaI); the oxide includes sodium oxide (Na₂O) and sodium peroxide (Na₂O₂); the hydroxide includes sodium hydroxide (sodium hydroxide (generic name): NaOH); the inorganic salt includes sodium hydride (NaH), sodium sulfide (Na₂S), sodium hydrogensulfide (sodium hydrosulfide) (NaHS), sodium silicate (Na₂SiO₃), trisodium phosphate (Na₃PO₄), sodium borate (Na₃BO₃), sodium borohydride (NaBH₄), and sodium tetrachloroaurate (Na[AuCl₄]); the organic acid salt includes sodium acetate (CH₃COONa) and sodium citrate; and the like. These sodium salts may be used in a single kind or a combination of plural kinds. In the present invention, as the single kind, sodium chloride is preferably used, and as the plural kinds sodium chloride and sodium citrate are preferably used. The amount of the sodium salt contained is not particularly limited, and the amount, in terms of a final concentration of the entire sodium ions contained in the cryopreservation solution of the present invention, is preferably from 0.01 to 5,000 mmol/L, more preferably from 0.1 to 1,000 mmol/L, and even more preferably from 1 to 300 mmol/L.

The "potassium salt" as used herein is not particularly limited so long as the potassium salt generates potassium ions when dissolved in a solvent, and an oxoacid salt, a halide, an oxide, a hydroxide, an inorganic salt, an organic acid salt, or the like can be used. For example, the oxoacid acid includes potassium nitrate (KNO₃), potassium sulfate (K₂SO₄), and potassium percarbonate (K₂CO₄); the halide includes potassium fluoride (KF), potassium hydrogenfluoride (KHF₂), potassium chloride (KCl), potassium bromide (KBr), and potassium iodide (KI); the oxide includes potassium peroxide (K₂O₂) and potassium oxide (K₂O); the hydroxide includes potassium hydroxide (KOH); the inorganic salt includes potassium hydride (KH), potassium sulfide (K₂S), potassium hydrogensulfide (potassium hydrosulfide: KSH), potassium permanganate (KMnO₄) potassium chromate (K₂CrO₄), and potassium chlorate (KClO₃); and the like. These potassium salts may be used in a single kind or a combination of plural kinds. In the present invention, potassium chloride is preferably used. The amount of the potassium salt contained is not particularly limited, and the amount, in terms of a final concentration of the entire potassium ions contained in the cryopreservation solution of the present invention, is preferably from 0.01 to 5,000 mmol/L, more preferably from 0.1 to 1,000 mmol/L, and even more preferably from 1 to 100 mmol/L.

The "hydrogencarbonate salt" as used herein is not particularly limited so long as the hydrogencarbonate salt generates hydrogencarbonate ions when dissolved in a solvent, and salts with various cations can be used. For example, the hydrogencarbonate salt includes ammonium hydrogencarbonate (NH₄HCO₃), potassium hydrogencarbonate (KHCO₃), calcium hydrogencarbonate (Ca(HCO₃)₂), sodium hydrogencarbonate (NaHCO₃), magnesium hydrogencarbonate (Mg(HCO₃)₂), and the like.

The "carbonate salt" as used herein is not particularly limited so long as the carbonate salt generates carbonate ions when dissolved in a solvent, and salts with various cations can be used. For example, the carbonate salt includes ammonium carbonate ((NH₄)₂CO₃), potassium carbonate (K₂CO₃), calcium carbonate (CaCO₃), sodium carbonate (Na₂CO₃), barium carbonate (BaCO₃), magnesium carbonate (MgCO₃), lithium carbonate (Li₂CO₃), copper(II) carbonate (CuCO₃), iron(II) carbonate (FeCO₂), silver(I) carbonate (Ag₂CO₃), and the like.

These hydrogencarbonate salt and/or carbonate salt may be used in a single kind or a combination of plural kinds. In the present invention, sodium hydrogencarbonate is preferably used. The amount of the hydrogencarbonate salt and/or carbonate salt contained is not particularly limited, and the amount, in terms of a final concentration of a total of the hydrogencarbonate ions and the carbonate ions contained in the cryopreservation solution of the present invention, is preferably from 0.01 to 1,000 mmol/L, more preferably from 0.1 to 500 mmol/L, and even more preferably from 1 to 100 mmol/L.

As the "saccharide" as used herein, a monosaccharide, an oligosaccharide, or a sugar alcohol can be used. For example, the monosaccharide includes glucose, galactose, fructose, mannose, xylose, and arabinose; the oligosaccharide includes trehalose, sucrose, maltose, lactose, and cellobiose; the sugar alcohol includes xylitol and sorbitol; and the like. These saccharides may be used in a single kind or a combination of plural kinds, and in the present invention, the saccharide is preferably at least one saccharide selected from the group consisting of glucose, galactose, fructose, mannose, xylose, and arabinose, and more preferably glucose. The amount of the saccharide contained is preferably from 0.01 to 100 g/L, more preferably from 0.1 to 100 g/L, and even more preferably from 0.25 to 50 g/L, of the cryopreservation solution.

The "cryoprotectant" as used herein includes dimethyl sulfoxide (DMSO), hydroxyethyl starch (HES), ethylene glycol, glycerol, and the like. In addition, the commercially available products include CP-1 [physiological saline containing HES 17.65% (W/V) and DMSO 14.71% (V/V), manufactured by KYOKUTO PHARMACEUTICAL INDUSTRIAL CO., LTD.]. These cryoprotectants may be used in a single kind or a combination of plural kinds. In the present invention, DMSO and hydroxyethyl starch are preferably used. The amount of the cryoprotectant contained is preferably from 0.01 to 50%, more preferably from 1 to 40%, and even more preferably from 3 to 30%, of the cryopreservation solution. In addition, in a case where DMSO and hydroxyethyl starch are used together as the cryoprotectants, it is preferable that they are contained in a total amount within the range mentioned above, and each concentration is such that the DMSO concentration is preferably from 0.01 to 50%, more preferably from 1 to 30%, and even more preferably from 2 to 15%, and the hydroxyethyl starch concentration is preferably from 0.01 to 50%, more preferably from 1 to 30%, and even more preferably from 2 to 15%.

In a preferred embodiment of the present invention, in addition to the essential components of the solution used in the method for cryopreservation of cells of the present invention mentioned above, the solution may further optionally contain a component selected from the group consisting of proteins, magnesium salts, and calcium salts.

The "protein" as used herein is not particularly limited, and the protein includes isolated proteins, or available recombinant proteins. For example, the protein includes proteins derived from blood. The protein derived from blood includes proteins derived from plasma, and proteins derived from sera, and concretely includes serum albumin, serum globulin, and the like. Also, the serum albumin includes human serum albumin or bovine serum albumin. The commercially available products include Buminate Injection 25% (25% human serum albumin, manufactured by Baxter Limited), and the like. These proteins may be used in a single kind or a combination of plural kinds. In the present invention, the protein is preferably human serum albumin. The amount of the protein contained is preferably from 0.01 to 50%, more preferably from 1 to 30%, and even more preferably from 2 to 15%, of the cryopreservation solution.

The "magnesium salt" as used herein is not particularly limited so long as the magnesium salt generates magnesium ions when dissolved in a solvent, and an oxoacid salt, a halide, an oxide, a hydroxide, an inorganic salt, an organic acid salt, or the like can be used. For example, the oxoacid acid includes magnesium nitrate (Mg(NO₃)₂), magnesium sulfate (MgSO₄), and magnesium percarbonate (MgCO₄); the halide includes magnesium fluoride (MgF₂), magnesium hydrogenfluoride (Mg(HF₂)₂), magnesium chloride (MgCl₂), magnesium bromide (MgBr₂), and magnesium iodide (MgI₂); the oxide includes magnesium peroxide (MgO₂) and magnesium oxide (MgO); the hydroxide includes magnesium hydroxide (Mg(OH)₂); the inorganic salt includes magnesium hydride (MgH₂), magnesium sulfide (MgS), magnesium hydrogensulfide (magnesium hydrosulfide: Mg(SH)₂), magnesium permanganate (Mg(MnO₄)₂), magnesium chromate (MgCrO₄), and magnesium chlorate (Mg(ClO₃)₂); and the like. These magnesium salts may be used in a single kind or a combination of plural kinds. In the present invention, magnesium chloride is preferably used. The amount of the magnesium salt contained is not particularly limited, and the amount, in terms of a final concentration of the entire magnesium ions contained in the cryopreservation solution of the present invention, is preferably from 0.01 from 10 mmol/L, and more preferably from 0.1 to 5 mmol/L.

The "calcium salt" as used herein is not particularly limited so long as the calcium salt generates calcium ions when dissolved in a solvent, and an oxoacid salt, a halide, an oxide, a hydroxide, an inorganic salt, an organic acid salt, or the like can be used. For example, the oxoacid salt includes calcium nitrate (Ca(NO₃)₂), calcium sulfate (CaSO₄), calcium percarbonate (CaCO₄); the halide includes calcium fluoride (CaF₂), calcium hydrogenfluoride (Ca(HF₂)₂), calcium chloride (CaCl₂), calcium bromide (CaBr₂), and calcium iodide (CaI₂); the oxide includes calcium peroxide (CaO₂) and calcium oxide (CaO); the hydroxide includes calcium hydroxide (Ca(OH)₂); the inorganic salt includes calcium hydride (CaH₂), calcium sulfide (CaS), calcium hydrogensulfide (calcium hydrosulfide: Ca(SH)₂), calcium permanganate (Ca(MnO₄)₂), calcium chromate (CaCrO₄), and calcium chlorate (Ca(ClO₃)₂); and the like. These calcium salts may be used in a single kind or a combination of plural kinds. In the present invention, calcium chloride is preferably used. The amount of the calcium salt contained is not particularly limited, and the amount, in terms of a final concentration of the entire calcium ions contained in the cryopreservation solution of the present invention, is preferably from 0.01 to 10 mmol/L, and more preferably from 0.1 to 5 mmol/L.

As a concentration ratio of the sodium salt to the potassium salt of the components of the cryopreservation solution used in the method of the present invention, the concentration ratio of the sodium ions to the potassium ions, sodium ions/potassium ions, is preferably from 1/1000 to 1000/1, more preferably from 1/100 to 100/1, even more preferably from 1/10 to 100/1, even more preferably from 1/1 to 100/1, and even more preferably from 10/1 to 50/1.

In addition, the cryopreservation solution of the present invention may further contain, in addition to the components mentioned above, a substance that does not have cytotoxicity, for example, a vitamin, an amino acid, or the like.

Further, the cryopreservation solution of the present invention may contain, in addition to the components mentioned above, phosphate ions, from the viewpoint of pH adjustment or exhibition of buffering actions. However, it is preferable that the constituent is made smaller, from the viewpoint of using the cells after thawing directly to a live body, or the cryopreservation solution may not contain the phosphate ions.

The cryopreservation solution of the present invention can be prepared by weighing each of a sodium salt, a potassium salt, a saccharide, a cryoprotectant, and a hydrogencarbonate salt and/or a carbonate salt in a necessary amount, and dissolving the entire components in pure water or physiological saline. Alternatively, the cryopreservation solution may be prepared by dissolving each of the components separately in pure water or physiological saline, and mixing each of the component solutions in necessary amounts. When the cryopreservation solution is prepared as described above, each of the components may be dissolved in pure water or physiological saline, and thereafter a bacterial elimination step (for example, a bacterial elimination filtration step) may be incorporated thereto. Further, a protein, a magnesium salt, or a calcium salt may be optionally prepared and added thereto in the same manner. Alternatively, several kinds of commercially available solutions containing a sodium salt, potassium salt, a saccharide, a cryoprotectant, and a hydrogencarbonate salt and/or a carbonate salt may be used in combination. A commercially available product, for example, BICARBON Infusion (manufactured by AJINOMOTO), Japanese Pharmacopoeia glucose solution (for example, manufactured by Nissin Pharmaceutical Co., LTD.), Japanese Pharmacopoeia sodium chloride solution (for example, manufactured by Otsuka Pharmaceutical Co., Ltd.), Japanese Pharmacopoeia potassium chloride solution (for example, manufactured by Otsuka Pharmaceutical Co., Ltd.), Japanese Pharmacopoeia sodium hydrogencarbonate solution (for example, manufactured by Otsuka Pharmaceutical Co., Ltd.), CP-1 (KYOKUTO PHARMACEUTICAL INDUSTRIAL CO., LTD.), or Buminate Injection 25% (25% human serum albumin, manufactured by Baxter Limited) may be mixed under sterile conditions so as to contain necessary concentrations for the present invention.

The osmotic pressure when the cryopreservation solution of he present invention is used is not particularly limited, and it is desired that the cryopreservation solution has an osmotic pressure ratio that would not give damages to the cells. For example, when used in the preservation of human lymphocytes, the osmotic pressure is preferably from 120 to 1,500 mOsm/L, and more preferably from 250 to 300 mOsm/L. In the adjustment of the osmotic pressure, any one of the essential components of the solution for use in the method for cryopreservation of cells of the present invention can be used.

The pH of the cryopreservation solution of the present invention is not particularly limited, and it is desired that the solution has a pH that would not give damages to the cells. For example, when used in the preservation of human lymphocytes, the pH is preferably from 3.0 to 10.0, and more preferably from 4.5 to 9.0. In the adjustment of the pH, any one of the essential components of the solution for use in the method for cryopreservation of cells of the present invention can be used.

A cryopreservation solution containing essential components including a sodium salt, a potassium salt, a saccharide, a cryoprotectant, and a hydrogencarbonate salt and/or a carbonate salt, and further optionally containing a component selected from the group consisting of proteins, magnesium salts and calcium salts, for use in the method for cryopreservation of cells of the present invention, as mentioned above, is also embraced by the present invention.

The cells which can be used in the method for cryopreservation of the present invention include various cells and cell population that are isolated from mammals including human, and cells induced therefrom. The various isolated cells include, for example, peripheral blood mononuclear cells (PBMCs), hemopoietic stem cells, umbilical cord blood mononuclear cells, hematocytes, lymphocytes, T cells, B cells, naive cells, memory cells, and the like, and the induced cells include lymphokine-activated killer cells (LAK cells), cytotoxic T cells (CTLs), tumor infiltrated lymphocytes (TIL), NK cells, and the like, each having cytotoxic activity, and the cells are not limited to these cells. These cells may be prepared by a known method.

The cell concentration when the intended cells are homogeneously suspended in a cryopreservation solution is not particularly limited, and the cell concentration may be properly determined depending upon the kinds of cells, use and applications of cells, sizes of cells, sizes of preservation vessels, preservation period, and the like, and the cell concentration is, for example, preferably from 1 × 10⁴ to 1 × 10⁹ cells/mL, more preferably from 1 × 10⁵ to 1 × 10⁹ cells/mL, and even preferably from 2 × 10⁵ to 5 × 10⁸ cells/mL. The preservation vessel is not particularly limited so long as the preservation vessel enables cryopreservation of the cells, and a cryopreservation bag, an aluminum protector, a miracle bag, a cryogenic vial or the like can be used.

In the present invention, the method for cryopreservation of cells refers to a method including the steps of suspending intended cells in the cryopreservation solution of the present invention to prepare a cell suspension, allowing the cell suspension to stand in a freezer held at a ultra-low temperature, thereby preserving the cell suspension in a frozen state. The ultra-low temperature as referred to herein is not particularly limited so long as it is a temperature at which the cell suspension is completely frozen, which may be usually at minus(-)60°C or lower. Also, a method for cryopreservation of cells using liquid nitrogen is also embraced by the present invention. Preferably, a cell suspension is preserved in a freezer filled with liquid nitrogen at a temperature of minus(-)70°C or lower. The cryopreservation time period according to the method of the present invention is not particularly limited, and the cryopreservation time period is, for example, from 5 minutes to 48 months, and preferably from one day to 12 months.

The cell suspension subjected to cryopreservation according to the method of the present invention is thereafter thawed, so that a thawed cell suspension can be further preserved in a non-frozen state. In other words, the present invention also provides a method for preservation of cells, **characterized in that** the method includes the steps of thawing the above-mentioned cell suspension subjected to cryopreservation (i.e. cryopreserved cell suspension), and further preserving a thawed cell suspension in a non-frozen state, in other words, a liquid suspension state. A method of thawing a cryopreserved cell suspension is not particularly limited, and the cell suspension in a frozen state may be thawed at a temperature of preferably from 0° to 40°C, and more preferably from 20° to 37°C. The preservation temperature after thawing is at a temperature of preferably from 0° to 40°C, and more preferably from 4° to 37°C. The preservation time period of the cell suspension in a non-frozen state is not particularly limited, and the preservation time period is, for example, from 0 to 18 days, preferably from 0 to 10 days, and more preferably from 0 to 3 days.

The cells subjected to cryopreservation according to the method of the present invention maintain high viability rates even after thawing, and it is also possible to further continue preserving the cells after thawing. In addition, the cells can be directly administered to a live body without going through a step of washing the cells after thawing.

### EXAMPLES

The present invention will be shown hereinbelow more specifically by means of the Examples, without intending to limit the scope of the present invention thereto. Here, in the present examples, "room temperature" means a temperature of from 20° to 30°C.

### Example 1 Studies on Preservation Solution

### (1) Preparation of Solutions to Be Studied

As preservation solutions for screening, the solutions as listed in Table 1 were prepared.

[Table 1]

**Table 1**

| Solution No. | |
|---|---|
| 1 | Physiological saline (comparative example: manufactured by Otsuka Pharmaceutical Co., Ltd.) |
| 2 | Calcium nitrate tetrahydrate 100 mg/L, magnesium sulfate 44.8 mg/L, sodium hydrogencarbonate 2 g/L, disodium hydrogenphosphate 800 mg/L, potassium chloride 400 mg/L, sodium chloride 6 g/L, aqueous solution containing glucose 2 g/L (final concentration: calcium ions: 0.42 mmol/L, magnesium ions: 0.37 mmol/L, hydrogencarbonate ions: 23.81 mmol/L, sodium ions: 137.78 mmol/L, phosphate ions: 5.64 mmol/L, potassium ions: 5.37 mmol/L; sodium ions/potassium ions = 25.66/1) |
| 3 | Aqueous solution resulting from removing calcium nitrate from Solution 2 (final concentration: magnesium ions: 0.37 mmol/L, hydrogencarbonate ions: 23.81 mmol/L, sodium ions: 137.78 mmol/L, phosphate ions: 5.64 mmol/L, potassium ions: 5.37 mmol/L; sodium ions/potassium ions =25.66/1) |
| 4 | Aqueous solution resulting from removing magnesium sulfate from Solution 2 (final concentration: calcium ions: 0.42 mmol/L, hydrogencarbonate ions: 23.81 mmol/L, sodium ions: 137.78 mmol/L, phosphate ions: 5.64 mmol/L, potassium ions: 5.37 mmol/L; sodium ions/potassium ions =25.66/1) |
| 5 | Aqueous solution resulting from removing sodium hydrogencarbonate from Solution 2, and changing a sodium chloride concentration to 7.38 g/L for osmotic pressure adjustment (final concentration: calcium ions: 0.42 mmol/L, magnesium ions: 0.37 mmol/L, sodium ions: 137.55 mmol/L, phosphate ions: 5.64 mmol/L, potassium ions: 5.37 mmol/L; sodium ions/potassium ions =25.61/1) |
| 6 | Aqueous solution resulting from removing disodium hydrogenphosphate from Solution 2, and changing a sodium chloride concentration to 6.6 g/L for osmotic pressure adjustment (final concentration: calcium ions: 0.42 mmol/L, magnesium ions: 0.37 mmol/L, hydrogencarbonate ions: 23.81 mmol/L, sodium ions: 136.75 mmol/L, potassium ions: 5.37 mmol/L; sodium ions/potassium ions =25.47/1) |
| 7 | Aqueous solution resulting from removing potassium chloride from Solution 2, and changing a sodium chloride concentration to 6.3 g/L for osmotic pressure adjustment (final concentration: calcium ions: 0.42 mmol/L, magnesium ions: 0.37 mmol/L, hydrogencarbonate ions: 23.81 mmol/L, sodium ions: 142.88 mmol/L, phosphate ions: 5.64 mmol/L) |

### (2) Preparation of Cells

Sixty milliliters of heparin-supplemented blood was collected from human healthy donor obtained with informed consent. The blood obtained was dispensed into a conical tube (manufactured by Becton Dickenson or Greiner) in an amount of about 15 mL each, and then centrifuged at 700 × g, room temperature for 20 minutes, to be separated into a plasma fraction, the supernatant after the centrifugation, and a cell fraction containing human peripheral blood mononuclear cells (PBMCs). The cell fraction containing PBMCs was diluted by filling up the conical tube to a volume of 20 mL with Dulbecco's PBS (manufactured by Invitrogen or manufactured by NISSUI PHARMACEUTICAL CO., LTD.). The diluted cell solution obtained was overlaid with 20 mL of Ficoll-Paque PREMIUM (manufactured by GE Healthcare Bioscience), and centrifuged at 700 × g, room temperature for 20 minutes. After the centrifugation, of the separated layers, the PBMC layer was collected with a pipette, and the tube was filled up to a volume of 30 mL with RPMI 1640 medium, and then centrifuged at 650 × g, 4°C for 10 minutes to remove the supernatant. Similarly, centrifugation procedures were sequentially carried out while lowering the centrifugal force stepwise to 600 × g, and then to 500 × g, and washing was repeated for a total of 3 times. The number of live cells of the PBMCs obtained and the viability rate (%) thereof were calculated using an automated blood cell counting instrument (nucleocounter, manufactured by Chemometec).

Using the PBMCs thus obtained, human T lymphocytes were prepared in accordance with the method described in WO 2009/019450. The medium was removed from the human T lymphocytes obtained, and the lymphocytes were suspended in physiological saline so as to have a cell concentration of 3 × 10⁸ cells/mL, to prepare a cell solution.

### (3) Screening of Cell Solution Subjected to Mild Temperature (37°C) Treatment

Forty microliters of each of the solutions to be studied which was prepared in Example 1-(1) and 10 µL of the cell solution prepared in Example 1-(2) were placed in a 1.5 mL plastic tube (manufactured by Treff) for each of the solutions to be studied, and mixed, to prepare a cell suspension. Thereafter, the cell suspension was allowed to stand in a thermostat set at 37°C for 3 hours.

### (4) Measurement of Cell Viability Activity in Accordance with MTT Assay

Each of the cell suspensions after the treatment described in Example 1-(3) was measured for the cell metabolism (respiration) as cell viability activity using the MTT assay described hereinbelow.

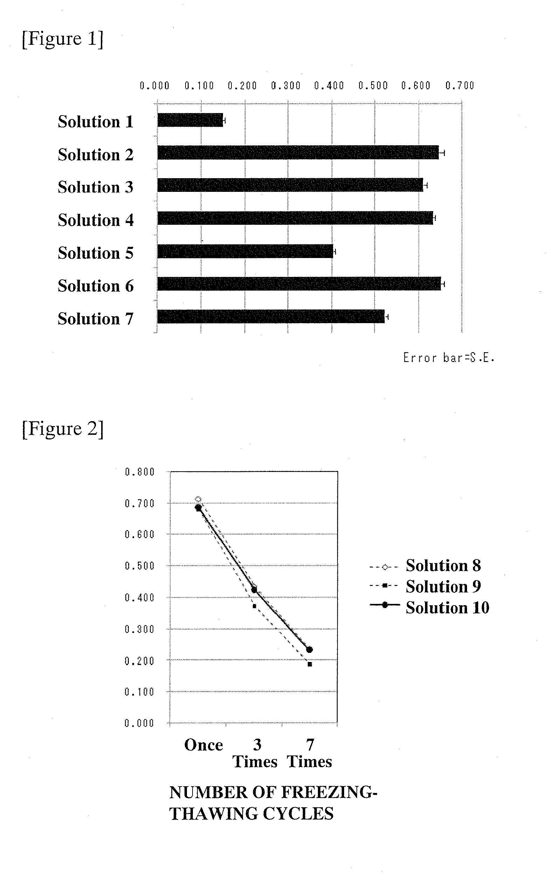

First, to 50 µL of each of the cell suspensions were added 50 µL of MTT [3-(4,5-dimethyl-2-thiazolyl)-2,5-diphenyltetrazolium bromide, manufactured by Nakalai Tesque] prepared to a concentration of 5 mg/mL with Dulbecco's PBS(-) (manufactured by NISSUI PHARMACEUTICAL CO., LTD.), and 400 µL of RPMI 1640 medium (manufactured by Wako Pure Chemicals Industries, Ltd.) containing 10% fetal bovine serum (manufactured by GIBCO) and a 1% penicillin-streptomycin mixed solution (manufactured by Nakalai Tesque), and mixed. Next, the mixed solution was seeded to 4 wells of a 96-well multi-plate (manufactured by Costar or manufactured by Becton Dickenson) in a volume of 100 µL each, and the cells were cultured under the conditions of 37°C and a carbon dioxide concentration of 5% for 90 minutes. After the termination of the culture, 100 µL of a hydrochloric acid-2-propanol test solution [prepared as prescribed in Fifteenth Revised Japanese Pharmacopoeia: solution prepared by mixing 2-propanol (special grade, manufactured by Nakalai Tesque) and hydrochloric acid (manufactured by Nakalai Tesque) in a ratio of 100:0.33] was added thereto, to completely dissolve the dye therein. The absorbance for the solution after dissolution was measured at a measuring wavelength of 570 nm and a control wavelength of 655 nm with a microplate absorption spectrophotometer (680XR, manufactured by Bio-Rad). The values of the absorbance obtained were regarded as cell viability activity (MTT activity values) of cell metabolism (respiration). The results are shown in Figure 1. In other words, Figure 1 is a diagram showing an average of the measurement values for the absorbance (measured at 4 wells) for each kind of solutions. In the figure, the axis of abscissas shows MTT activity values. In addition, in the figure, "Error Bar = S.E." shows a standard error of the measurement values for 4 wells.

It is clear from Figure 1 that as compared to Solution 2 containing 7 kinds of components, Solution 5 without containing sodium hydrogencarbonate and Solution 7 without containing potassium chloride had lowered MTT activity values, so that it was clarified that these components are essential in stabilization of the cells. On the other hand, in the solution without containing calcium nitrate, magnesium sulfate, or disodium hydrogenphosphate (Solution 3, Solution 4, or Solution 6, respectively), the MTT activity value was not lowered, so that it was clarified that these components are not essential in stabilization of the cells.

Similar tests were conducted for all 40 kinds of components that are elucidated in RPMI 1640 medium in various combinations at various concentrations, and further tests of one kind or a combination of several kinds of components of DMSO and hydroxyethyl starch as cryopretectants, and albumin as the protein were conducted. Screening was carried out for a total of 100 or more kinds of solutions, and which of the components are essential in stabilization of cells was comparatively studied. As a result, of the 40 kinds of components mentioned above, it was elucidated that four components, namely the sodium salts, the potassium salts, the hydrogencarbonate salts and/or carbonate salts, and the saccharides are necessary and sufficient.

### Example 2 Influences to Freezing-Thawing

### (1) Preparation of Solutions to Be Studied

As preservation solutions for studies on stability against freezing-thawing procedures, 3 kinds of solutions shown in Table 2 were prepared.

[Table 2]

**Table 2**

| Solution No. | |
|---|---|
| 8 | RPMI 1640 medium (comparative example: manufactured by Wako Pure Chemicals) |
| 9 | Physiological saline (comparative example: manufactured by Otsuka Pharmaceutical Co., Ltd.) (final concentration: sodium ions: 154.00 mmol/L) |
| 10 | Aqueous solution containing sodium chloride 6.6 g/L, potassium chloride 0.40 g/L, sodium hydrogencarbonate 2.0µ/L, glucose 2.0 g/L (final concentration: hydrogencarbonate ions: 23.81 mmol/L, sodium ions: 136.75 mmol/L, potassium ions: 5.37 mmol/L; sodium ions/potassium ions=25.47/1) |

### (2) Preparation of Cells to Be Tested

A cell solution was prepared in the same manner as in Example 1-(2), provided that the cells were suspended in physiological saline so as to have a concentration of 6 × 10⁸ cells/mL.

### (3) Preservation of Cells (Freezing-Thawing Treatment of Cell Solution)

Forty microliters of each of the solutions to be studied which was prepared in Example 2-(1), 10 µL of the cell solution prepared in Example 2-(2), 34 µL of CP-1 (manufactured by KYOKUTO PHARMACEUTICAL INDUSTRIAL CO., LTD.) (amount of cryoprotectant contained in the cell suspension: 34%), and 16 µL of Buminate Injection 25% (25% human serum albumin, manufactured by Baxter Limited) [amount of proteins contained in the cell suspension: 4%(W/V)] were placed in a cryogenic vial (manufactured by NALGENE) for each of the solutions to be studied, and mixed, to prepare a cell suspension. Here, 3 vials each of the cell suspensions were prepared. Thereafter, the cell suspension was allowed to stand in an ultra-cryostat set at minus(-)85°C for 18 hours or more, to allow the cell suspension to be completely frozen. After confirmation of being completely frozen, the cell suspension was transferred to a thermostat set at 37°C and thawed over 5 minutes. The procedures were repeated once, 3 times, or 7 times, and the products were used in the subsequent measurement.

### (4) Measurement of Cell Viability Activity in Accordance with MTT Assay

For each of the cell suspensions after the treatment described in Example 2-(3), the cell viability activity was measured using the MTT assay in the same manner as in Example 1-(4). The results are shown in Figure 2. In other words, Figure 2 is a diagram showing an average of the absorbance of the measurement values (measured at 4 wells) for each kind of solutions and the number of freezing-thawing cycles. In the figure, the axis of abscissas shows the number of freezing-thawing cycles, and the axis of ordinates shows MTT activity values. In addition, the results of Solution 8 are shown by a dotted line connecting open squares. The results of Solution 9 are shown by a dotted line connecting solid squares. The results of Solution 10 are shown by a solid line connecting solid circles.

As is evident from Figure 2, Solution 10 containing only 4 components of sodium chloride, potassium chloride, sodium hydrogencarbonate, and glucose was clarified to have the equivalent level of cell viability activity to that of Solution 8 (RPMI 1640 medium) containing 40 kinds of numerous components, in any cases of freezing-thawing cycles of once, 3 times, and 7 times. On the other hand, physiological saline had drastically lowered activity by repeating the freezing-thawing procedures, so that it was clarified that this component alone is insufficient.

### Example 3 Stability Against Mild Temperature State (37°C) During Thawing and Before or After Thawing of Cells

### (1) Preparation of Solutions to Be Studied

A solution similar to the solution described in Example 2-(1) was prepared.

### (2) Preparation of Cells to Be Tested

A cell solution was prepared in the same manner as in Example 2-(2).

### (3) Mild Temperature (37°C) Treatment of Cell Solution

Forty microliters of each of the solutions to be studied which was prepared in Example 3-(1), 10 µL of the cell solution prepared in Example 3-(2), 34 µL of CP-1 (manufactured by KYOKUTO PHARMACEUTICAL INDUSTRIAL CO., LTD.), and 16 µL of Buminate Injection 25% (25% human serum albumin, manufactured by Baxter Limited) were placed in a cryogenic vial (manufactured by NALGENE) for each of the solutions to be studied, and mixed, to prepare a cell suspension. Thereafter, the cell suspension was subjected to freezing-thawing in the same manner as in Example 2-(3), provided that the freezing-thawing cycle was carried out once, and that each of the cell solutions after thawing was allowed to stand in a thermostat set at 37°C for 3 hours. Here, the content of the cryoprotectant and the content of the protein in the cell suspension are the same as those in Example 2-(3).

### (4) Measurement of Cell Viability Activity According to MTT Assay

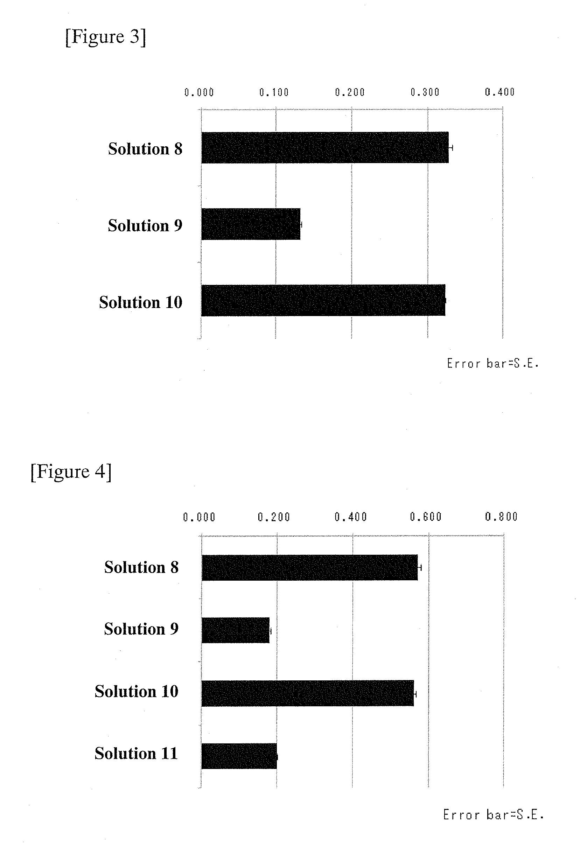

For each of the cell suspensions after the treatment described in Example 3-(3), the cell viability activity was measured using the MTT assay in the same manner as in Example 1-(4). The results are shown in Figure 3. In other words, Figure 3 is a diagram showing an average of the absorbance of the measurement values (measured at 4 wells) for each kind of solutions. In the figure, the axis of abscissas shows MTT activity values.

Using cells prepared in the same manner as in Example 1-(2) from blood of human healthy donors different from those in Example 1-(2), obtained with informed consent, the solution described in Example 2-(1) and the following Solution 11 were prepared, and similar studies were made, provided that 16 µL of physiological saline (manufactured by Otsuka Pharmaceutical Co., Ltd.) was mixed with each of the cell suspensions in place of the Buminate Injection used in Example 3-(3). Physiological saline is required for regulating an osmotic pressure, and is also used in the negative control so that the addition is required. By the addition, the ion concentration increases, but it is considered that there are no special stabilization effects caused thereby.
- Solution 11: Aqueous solution containing 6.6 g/L sodium chloride, 0.40 g/L potassium chloride, and 2.0 g/L glucose (final concentration: sodium ions: 112.94 mmol/L, potassium ions: 5.37 mmol/L; sodium ions/potassium ions=21.03/1)

The results are shown in Figure 4. In other words, Figure 4 is a diagram showing an average of the absorbance of the measurement values (measured at 4 wells) for each kind of solutions. In the figure, the axis of abscissas shows MTT activity values.

As is evident from Figures 3 and 4, even when the cell solution after freezing and thawing was allowed to stand in a mild temperature state for a long period of time, Solution 10 containing 4 components of sodium chloride, potassium chloride, sodium hydrogencarbonate, and glucose, and a cryoprotectant, and a protein was clarified to have the equivalent level of activity to that of Solution 8 (RPMI 1640 medium) containing 40 kinds of numerous components, and a cryoprotectant and a protein, used in a conventional method. On the other hand, Solution 9 (physiological saline) and Solution 11 (Solution 10 without containing sodium hydrogencarbonate) had drastically lowered activity when the cell solutions were allowed to stand after freezing and thawing in a mild temperature state for a long period of time, so that it was clarified that this component alone is insufficient. In addition, the preserved cells showed the same tendencies in Solutions 8 to 10 of Figures 3 and 4, so that it was clarified that the preserved cells are not affected by the differences in donors.

### Example 4: Ability or Inability 1 of Being Constituted for Medicament with Proven Achievements in Injections

### (1) Preparation of Solutions to Be Studied

Solutions shown in Table 3 were prepared in order to study on whether or not similar results are obtained even when the solutions having high cell viability activity by freezing and thawing in Examples 2 and 3 are constituted with a solution which was registered as a medicament in Japan and has proven achievements in injections.

[Table 3]

**Table 3**

| Solution No. | |
|---|---|
| 12 | RPMI 1640 medium (reagent: manufactured by Wako Pure Chemical Industries, Ltd.) |
| 13 | Physiological saline (medicament: manufactured by Otsuka Pharmaceutical Co., Ltd.) (final concentration: sodium ions: 154.00 mmol/L) |
| 14 | Solution prepared by mixing BICARBON (medicament: manufactured by AJINOMOTO) and Japanese Pharmacopoeia 5% glucose injection "Nissin" (medicament: Nissin Pharmaceutical Co., LTD.) in a ratio of 25:1 (final concentration: hydrogencarbonate ions: 24 mmol/L, sodium ions: 130 mmol/L, potassium ions: 4 mmol/L, saccharide content: 1.9 g/L; sodium ions/potassium ions =33.75/1) |

### (2) Preparation of Cells to Be Tested

A cell solution was prepared in the same manner as in Example 2-(2).

### (3) Freezing and Thawing Treatment of Cell Solution

Forty microliters of each of the solutions to be studied which was prepared in Example 4-(1), 10 µL of the cell solution prepared in Example 4-(2), 34 µL of CP-1 (manufactured by KYOKUTO PHARMACEUTICAL INDUSTRIAL CO., LTD.), and 16 µL of Buminate Injection 25% (25% human serum albumin, manufactured by Baxter Limited) were placed in a cryogenic vial (manufactured by NALGENE) for each of the solutions to be studied, and mixed, to prepare a cell suspension. Here, 3 vials each of the cell suspensions were prepared. Thereafter, the freezing and thawing procedures were carried out in the same manner as in Example 2-(3), and those in which the freezing and thawing were repeated once, 3 times, and 7 times were used in the subsequent measurements. The content of the cryoprotectant and the content of the protein in the cell suspension are the same as in Example 2-(3).

### (4) Measurement of Cell Viability Activity in Accordance with MTT Assay

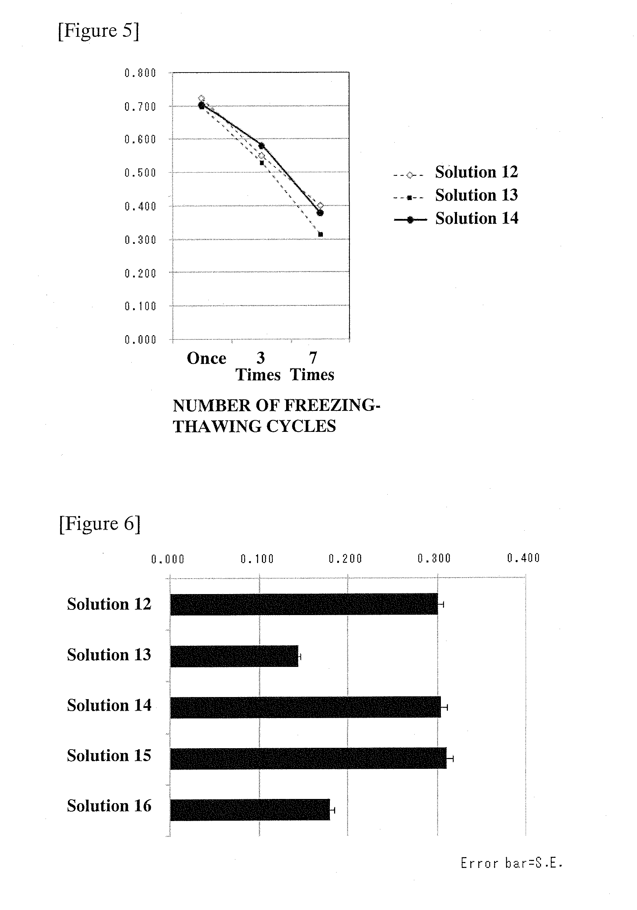

For each of the cell suspensions after the treatment described in Example 4-(3), the cell viability activity was measured using the MTT assay in the same manner as in Example 1-(4). The results are shown in Figure 5. In other words, Figure 5 is a diagram showing an average of the absorbance of the measurement values (measured at 4 wells) for each kind of solutions and the number of freezing-thawing cycles. In the figure, the axis of abscissas shows the number of freezing-thawing cycles, and the axis of ordinates shows MTT activity values. In addition, the results of Solution 12 are shown by a dotted line connecting open squares. The results of Solution 13 are shown by a dotted line connecting solid squares. The results of Solution 14 are shown by a solid line connecting solid circles.

As is evident from Figure 5, Solution 14 had an equivalent level of activity to that of Solution 12 (RPMI 1640 medium). From the results, since the preservation solution is constituted by using a medicament with proven achievements on injections, containing the 4 components of sodium chloride, potassium chloride, sodium hydrogencarbonate, and glucose, it was clarified that a preservation solution having an equivalent level of properties to a preservation solution obtained by using a solution containing numerous quasi-drugs used in conventional methods is obtained.

### Example 5: Ability or Inability 2 of Being Constituted for Medicament with Proven Achievements in Injections

### (1) Preparation of Solutions to Be Studied

The solutions described in Example 4-(1) and solutions of Table 4 were prepared.

[Table 4]

**Table 4**

| Solution No. | |
|---|---|
| 15 | Solution prepared by mixing BICARBON (medicament: manufactured by AJINOMOTO) and Japanese Pharmacopoeia 5% glucose injection "Nissin" (medicament: Nissin Pharmaceutical Co., LTD.) in a ratio of 24:1 (final concentration: hydrogencarbonate ions: 24 mmol/L, sodium ions: 130 mmol/L, potassium ions: 4 mmol/L, saccharide content: 1.9 g/L; sodium ions/potassium ions =33.75/1) |
| 16 | BICARBON (medicament: manufactured by AJINOMOTO) (final concentration: hydrogencarbonate ions: 25 mmol/L, sodium ions: 135 mmol/L, potassium ions: 4 mmol/L, sodium ions/potassium ions =33.75/1) |

### (2) Preparation of Cells to Be Tested

A cell solution was prepared in the same manner as in Example 2-(2).

### (3) Mild Temperature (37°C) Treatment of Cell Solution

Forty microliters of each of the solutions to be studied which was prepared in Example 5-(1), 10 µL of the cell solution prepared in Example 5-(2), 34 µL of CP-1 (manufactured by KYOKUTO PHARMACEUTICAL INDUSTRIAL CO., LTD.), and 16 µL of Buminate Injection 25% (25% human serum albumin, manufactured by Baxter Limited) were placed in a cryogenic vial (manufactured by NALGENE), and mixed, to prepare a cell suspension. Thereafter, the cell suspension was subjected to freezing-thawing in the same manner as in Example 2-(3), provided that the freezing-thawing cycle was carried out once, and that each of the cell solutions after thawing was allowed to stand in a thermostat set at 37°C for 3 hour. Here, the content of the cryoprotectant and the content of the protein in the cell suspension are the same as those in Example 2-(3).

### (4) Measurement of Cell Viability Activity According to MTT Assay

For each of the cell suspensions after the temperature treatment described in Example 5-(3), the cell viability activity was measured using the MTT assay in the same manner as in Example 1-(4). The results are shown in Figure 6. In other words, Figure 6 is a diagram showing an average of the measurement values for the absorbance (measured at 4 wells) for each kind of solutions. In the figure, the axis of abscissas shows MTT activity values.

As is evident from Figure 6, Solutions 14 and 15 had the equivalent level of activity to that of Solution 12 (RPMI 1640 medium). From the results, since the preservation solution is constituted by using a medicament with proven achievements in injections, containing the 4 components of sodium chloride, potassium chloride, sodium hydrogencarbonate, and glucose, it was clarified that properties of an equivalent level to a preservation solution obtained by using a solution containing numerous quasi-drugs used in conventional methods are obtained.

### INDUSTRIAL APPLICABILITY

According to the present invention, a method for cryopreservation of cells capable of maintaining a high viability rate after thawing, and a cryopreservation solution used in the cryopreservation are provided. In addition, by using the method for cryopreservation, the cells can be preserved in a state of maintaining a high viability rate even after thawing, and also maintaining their physiological functions; further, a cell washing step after freezing and thawing is unnecessary, thereby making it very useful in the fundamental researches of cells and the application studies on the medical treatment.

## Claims

1. A method for cryopreservation of cells **characterized by** the use of a solution comprising, as essential components, a sodium salt, a potassium salt, a saccharide, a cryoprotectant, and a hydrogencarbonate salt and/or a carbonate salt, the solution further optionally comprising a component selected from the group consisting of proteins, magnesium salts, and calcium salts.

2. The method according to claim 1, wherein the saccharide is at least one saccharide selected from the group consisting of glucose, galactose, fructose, mannose, xylose, and arabinose.

3. The method according to claim 1 or 2, wherein the cryoprotectant is at least one protectant selected from the group consisting of dimethyl sulfoxide, hydroxyethyl starch, ethylene glycol, and glycerol.

4. The method according to any one of claims 1 to 3, wherein the protein is a protein derived from blood.

5. A method for preservation of cells **characterized in that** the method comprises thawing a cell solution subjected to cryopreservation in accordance with the method as defined by any one of claims 1 to 4, and further preserving the cell solution in a liquid suspension state.

6. A cryopreservation solution of cells, **characterized in that** the cryopreservation solution comprises, as essential components, a sodium salt, a potassium salt, a saccharide, a cryoprotectant, and a hydrogencarbonate salt and/or a carbonate salt, and that the solution further optionally comprises a component selected from the group consisting of proteins, magnesium salts, and calcium salts.
